# EUROPEAN PATENT SPECIFICATION

(11) **EP 1 753 872 B1**
(45) Date of publication and mention of the grant of the patent: **29.01.2014**
(21) Application number: 05763367.9
(22) Date of filing: 08.06.2005
(51) Int. Cl.: C12Q 1/00, G01N 33/543, G01N 33/62

(54) **ELECTROCHEMICAL UREA SENSORS AND METHODS OF MAKING THE SAME**
ELEKTROCHEMISCHE HARNSTOFFSENSOREN UND VERFAHREN ZU IHRER HERSTELLUNG
DETECTEURS ELECTROCHIMIQUES D'UREE ET LEURS PROCEDES DE FABRICATION

(30) Priority: 09.06.2004 US 864908
(43) Date of publication of application: 21.02.2007
(73) Proprietor: Instrumentation Laboratory Company, Bedford, MA 01730 (US)
(72) Inventor: SHIN, Jungwon, Dongjak-gu, Seoul (KR); COSOFRET, Vasile, Acton, MA 01720 (US)
(74) Representative: Kirkham, Nicholas Andrew
(86) International application number: PCT/US2005/020120
(87) International publication number: WO 2005/123938

(56) References cited:
- WO-A-02/097419
- GB-A- 2 194 843
- US-A- 4 713 165
- US-A- 5 212 050
- US-A- 5 326 449
- US-A- 5 798 030

## Description

### Technical Field

The present invention is related to the field of electrochemical sensors, particularly to sensors that measure urea in analytical samples.

### Background Information

Researchers and clinicians often need to measure the concentration of various analytes in biological samples. These analytes include dissolved gases (e.g. carbon dioxide), ions (e.g. hydrogen, sodium, potassium, calcium, lithium, ammonium, and magnesium), and biologically active molecules (e.g. urea). In many cases, the biological sample is a body fluid (*e.g*., blood, serum, plasma, cerebro-spinal fluid, saliva, and/or urine) taken from a patient during an office visit or while undergoing surgery. Proper diagnosis and treatment often depend upon the accuracy of these measurements and the speed with which they are obtained.

An electrochemical sensor system is an analytical tool that can be used to measure the concentration of an analyte in a biological sample. The electrochemical sensor contains a physical transducer, such as a metal electrode, separated from the analytical sample by at least one semi-permeable membrane. Enzyme sensors, which detect and/or measure biological metabolites (such as carbohydrates, peptides, lipids, nucleotides, or urea, for example) in an analytical sample, include an enzymatic layer that covers the semi-permeable membrane. The enzymatic layer contains at least one enzyme that reacts with the analyte of interest to produce a chemical byproduct, which can migrate through the semi-permeable membrane and I be detected by the electrode. An example of an enzyme sensor is a urea sensor, which can contain an enzyme, such as urease, that hydrolyzes urea to form ammonium ions.

Existing urea sensors generally have a short lifespan compared to other types of sensors (*e.g*., ion sensors). This is due to the fact that the enzymatic layer of existing urea sensors is in direct contact with the analytical sample, which allows the : enzyme and other components of the membrane to leach into the sample and/or contaminants in the sample to degrade or destroy the enzyme in the enzyme layer.

US 4,713,165 A discloses a sensor having an ion-selective electrode.

US 5,326,449 A discloses a composite membrane for a sensor device for measuring the concentration of an analyte in solution.

GB 2194843 A discloses an enzyme electrode membrane and a method of making the same. WO 02/097419 A discloses a cross-linked enzyme matrix.

### Summary of the Invention

The present invention provides a urea sensor in which the enzymatic layer is not in direct contact with the sample, which results in a sensor that allows for multiple and continuous determinations of urea concentration in samples. In addition, the urea sensor of the present invention is amenable to miniaturization and can be incorporated into sensor cards containing other miniaturized electrochemical sensors used for continuous, long-term analysis of multiple analytes without compromising the overall useful life of the sensor card.

In general, in one aspect, the present invention features a sensor for detecting urea in a sample that includes an electrode, an enzymatic layer, and an outer diffusional layer. The outer diffusional layer is permeable to urea and is disposed between the enzymatic layer and the sample. By preventing direct contact between the enzymatic layer and the sample, the outer diffusional layer inhibits degradation and/or loss of the enzyme, thus extending the life of the urea sensor. The urea sensor can be used to determine the urea concentration of blood, serum, plasma, cerebro-spinal fluid, saliva, and/or urine samples, for example.

Embodiments of this aspect of the invention may include the following features. The electrode may be a metal electrode, such as a silver/silver chloride electrode. The enzymatic layer may contain an enzyme, such as urease, that catalyzes the hydrolysis of urea to ammonium ions. The enzymatic layer may also contain one or more enzyme stabilizers, including, for example, polypeptides (*e.g*., glutathione) and inert proteins (*e.g*., bovine serum albumin). In a particular embodiment, the enzymatic layer includes urease cross-linked to glutathione. In other embodiments, the enzymatic layer includes one or more inert proteins, such as bovine serum albumin, cross-linked to at least one of urease and glutathione. The outer diffusional layer can include a polymer, such as polyurethane, a poly(tetrafluoroethylene) ionomer, poly-(2-hydroxymethyl methacrylate), polyvinyl chloride, carboxylated polyvinyl chloride, hydroxylated polyvinyl chloride, polycarbonate, cellulose acetate, and mixtures thereof.

The urea sensor further includes an ion-selective membrane disposed between the electrode and the enzymatic layer. For example, the ion-selective membrane can be an ammonium ion-selective membrane that includes a polymer matrix in which an ammonium-selective ionophore is disposed. The polymer matrix can include, for example, polyvinyl chloride, carboxylated polyvinyl chloride, hydroxylated polyvinyl chloride, polyurethane, poly(tetrafluoroethylene), poly(methyl methacrylate), silicone rubber, and mixtures thereof. Examples of suitable ammonium ion-selective ionophores include nonactin, monactin, dinactin, trinactin, tetranactin, narasin, benzocrown ethers, cyclic depsipeptides, and mixtures thereof.

In another aspect, the present invention features a sensor card that includes one or more sensors described above disposed in or on a substrate.

In yet another aspect, the present invention provides methods for forming a sensor described above. In one approach, the method includes the steps of providing an electrode, applying to at least one surface of the electrode an enzymatic layer, and applying an outer diffusional layer over the enzymatic layer. In another approach, the method includes the steps of providing an electrode, applying to at least one surface of the electrode an ion-selective membrane, applying to the ion-selective membrane an enzymatic layer, and applying an outer diffusional layer over the enzymatic layer.

The foregoing and other objects, aspects, features, and advantages of the invention will become more apparent from the following description and from the claims.

### Brief Description of the Drawings

In the drawings, like reference characters generally refer to the same parts throughout the different views. Also, the drawings are not necessarily to scale, emphasis instead generally being placed upon illustrating the principles of the invention.
Fig. 1 is a schematic diagram of the components of an embodiment of an electrochemical sensor system according to the invention, including a sensor cartridge with an electrode card and sample inlet, a peristaltic pump, and a microprocessor.
Fig. 2 illustrates a frontal view of an embodiment of an electrode card according to the invention.
Fig. 3 illustrates a cross sectional view of an embodiment of an ion-selective electrode (ISE) according to the invention.
Fig. 4 illustrates a cross sectional view of an embodiment of a carbon dioxide (CO₂) electrode according to the invention.
Fig. 5 illustrates a cross sectional view of an embodiment of an enzyme electrode according to the invention.
Fig. 6 is a table containing examples of polymeric membrane components and their respective weight percentages for four different ISEs.
Fig. 7 is a graphical representation of the chronopotentiometric responses of an electrode card that includes a sodium ISE with a polymeric membrane containing high molecular weight polyvinyl chloride (HMW-PVC), with measurements taken before and after assaying a sodium-containing sample contaminated with 10 mg/dL thiopental sodium (thiopental).
Fig. 8 is a graphical representation of the chronopotentiometric responses of an electrode card that includes five sodium ISEs with polymeric membranes containing carboxylated polyvinyl chloride (PVC-COOH) according to the invention, with measurements taken before and after assaying a sodium-containing sample contaminated with 10 mg/dL thiopental.
Fig. 9 is a graphical representation of the chronopotentiometric responses of an electrode card that includes a potassium ISE with a polymeric membrane containing HMW-PVC, with measurements taken before and after assaying a potassium-containing sample contaminated with 10 mg/dL thiopental.
Fig. 10 is a graphical representation of the chronopotentiometric responses of an electrode card that includes five potassium ISEs with polymeric membranes containing PVC-COOH according to the invention, with measurements taken before and after assaying a potassium-containing sample contaminated with 10 mg/dL thiopental.
Fig. 11 is a graphical representation of the chronopotentiometric responses of an electrode card that includes a calcium ISE with a polymeric membrane containing HMW-PVC, with measurements taken before and after assaying a calcium-containing sample contaminated with 10 mg/dL thiopental.
Fig. 12 is a graphical representation of the chronopotentiometric responses of an electrode card that includes five calcium ISEs with polymeric membranes containing PVC-COOH according to the invention, with measurements taken before and after assaying a calcium-containing sample contaminated with 10 mg/dL thiopental.
Fig. 13 is a graphical representation of the chronopotentiometric responses of an electrode card that includes a pH electrode with a polymeric membrane containing HMW-PVC, with measurements taken before and after assaying a pH buffered sample contaminated with 10 mg/dL thiopental.
Fig. 14 is a graphical representation of one of the chronopotentiometric responses of an electrode card that includes five pH electrodes with polymeric membranes containing PVC-COOH according to the invention, with measurements taken before and after assaying a pH buffered sample contaminated with 10 mg/dL thiopental.
Fig. 15 is a graphical representation of the chronopotentiometric responses of an electrode card that includes a CO₂ electrode with a polymeric membrane containing HMW-PVC, with measurements taken before and after assaying a sample contaminated with 10 mg/dL thiopental.
Fig. 16 is a graphical representation of the chronopotentiometric responses of an electrode card that includes four CO₂ electrodes with polymeric membranes containing PVC-COOH according to the invention, with measurements taken before and after assaying a sample contaminated with 10 mg/dL thiopental.
Fig. 17 is a graphical representation of the bulk membrane resistance for an ISE with a polymeric membrane containing HMW-PVC.
Fig. 18 is a graphical representation of the bulk membrane resistance for an ISE with a polymeric membrane containing PVC-COOH.
Fig 19 is a graphical representation comparing sodium concentration values in whole blood samples determined by a sodium ISE with a polymeric membrane containing HMW-PVC against those determined by an electrode card including five sodium ISEs with polymeric membranes containing PVC-COOH according to the invention.
Fig. 20 is a graphical representation comparing potassium concentration values in whole blood samples determined by a potassium ISE with a polymeric membrane containing HMW-PVC against those determined by an electrode card including five potassium ISEs with polymeric membranes containing PVC-COOH according to the invention.
Fig. 21 is a graphical representation of the negative drift in potential over time of a sodium ISE with a polymeric membrane containing PVC-COOH according to the invention.
Fig. 22 is a graphical representation of the response of a urea sensor according to the invention to two different calibration solutions.
Fig. 23 is a graphical representation comparing urea concentration values in plasma samples determined by a commercially available clinical chemistry analyzer against those in corresponding whole blood samples determined by an electrode card including a urea sensor according to the invention.

### Description

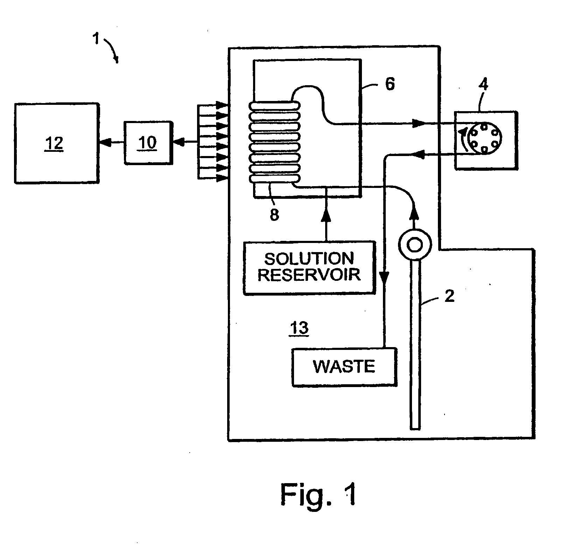

An electrochemical sensor according to the invention can be incorporated into an electrochemical sensor system. Referring to Figure 1, in one embodiment according to the invention, the electrochemical sensor system 1 has an inlet 2 where the biological sample is introduced into the electrochemical sensor system 1. A peristaltic pump 4 moves a sample, such as a body fluid sample, through the inlet 2 and into an electrode card 6. The electrode card 6 contains one or more electrodes 8 that detect and measure analytes of interest in the sample. An electrical interface 10 connects the electrode card 6 to a microprocessor 12. Signals from the electrode card 6 pass to the microprocessor 12 to allow for storage and display of the signals. Signals from the microprocessor 12 pass to the electrode card 6 to allow for control over measurement conditions, such as the polarization voltage of an electrode. In one embodiment according to the invention, the sample inlet 2 and the electrode card 6 are contained within a disposable cartridge 13, which can be detached from the remaining elements of the electrochemical sensor system 1 and replaced after use.

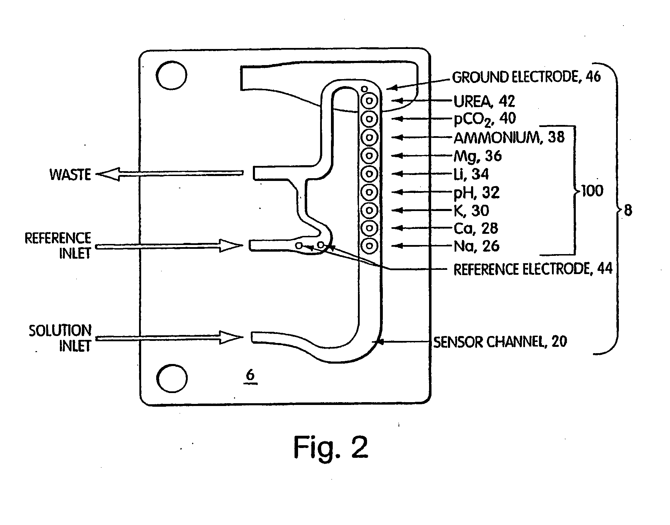

Referring to Figure 2, in one embodiment according to the invention, the electrode card 6 includes a rigid, substantially rectangular card made of polyvinyl chloride (PVC). A channel 20 is located within the electrode card 6, through which a biological sample or a reference solution can flow. One or more electrodes 8 can be embedded within the channel 20. When a sample is passed through the electrode card 6, it flows through the channel 20 and over the electrodes 8, allowing for detection and/or measurement of the analyte(s) of interest.

Referring to Figure 2, the electrodes 8 that can be incorporated into the electrode card 6 include ion-selective electrodes (ISEs) 100, electrodes for analyzing dissolved gases (gas electrodes), and electrodes which use an enzyme-based detection system (enzyme electrodes). For example, the electrodes may detect sodium 26, calcium 28, potassium 30, pH 32, lithium 34, magnesium 36, ammonium 38, carbon dioxide 40, and urea 42.

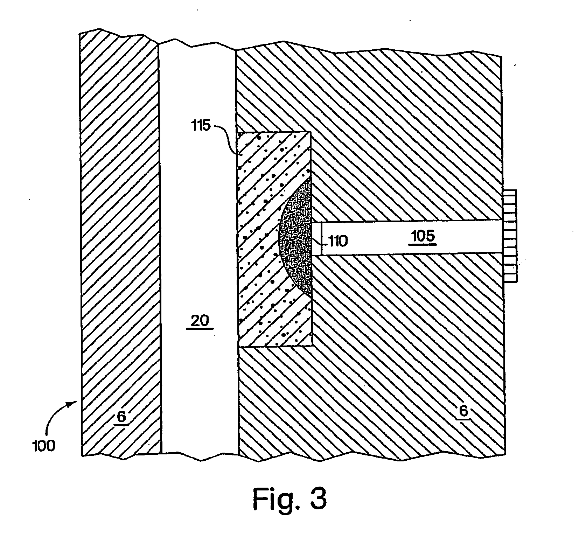

Referring to Figure 3, in one embodiment according to the invention, an ISE 100 comprises a metal element 105, an inner solution layer 110, and a polymeric membrane 115. The metal element 105 is embedded in the PVC of an electrode card 6, and the inner solution layer 110 covers the exposed end of the metal element 105. The inner solution layer 110 may contain, for example, 2-[N-morpholino]ethanesulfonic acid (MES) buffer. The polymeric membrane 115 is an ion-selective membrane that separates the inner solution layer 110 from an analytical sample (for example, a body fluid sample) that passes through the channel 20 in the electrode card 6. The composition of the polymeric membrane 115 determines the selectivity of the ISE 100 for a particular ion. In a particular embodiment according to the invention, PVC-COOH is a component of the polymeric membrane 115.

Referring back to Figure 2, to measure the concentration of an ion in an analytical sample, an ISE 100 must work in tandem with a reference electrode 44. If the ion that the ISE 100 is designed to detect is present in the analytical sample, an electrical potential is generated across the polymeric membrane 115 that depends on the difference between the concentration of the analyte in the inner solution layer 110, illustrated in Figure 3, and its concentration in the analytical sample. The difference in electrical potential between the ISE 100 and the reference electrode 44 is directly proportional to the change in the logarithm of the concentration of the measured ion in the analytical sample.

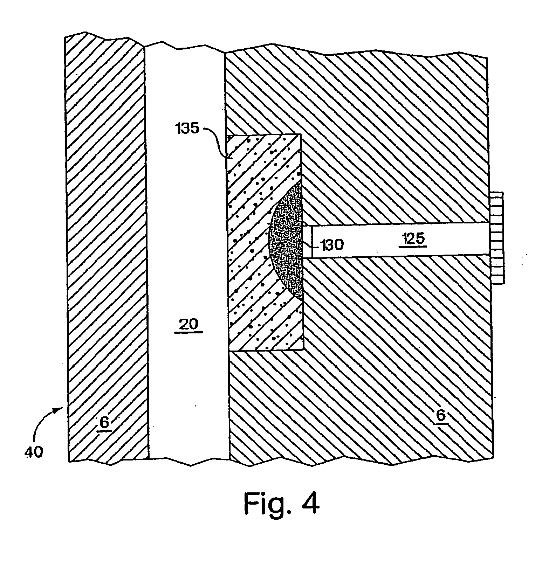

Referring to Figure 4, in one embodiment according to the invention, a carbon dioxide (CO₂) electrode 40, one type of gas electrode, comprises a metal element 125, an inner solution layer 130, and a polymeric membrane 135. The CO₂ electrode 40 is functionally similar to an ISE 100, except that the inner solution layer 130 of the CO₂ electrode 40 is bicarbonate buffer. Referring to Figure 2, unlike an ISE 100, the CO₂ electrode 40 must work in tandem with a pH electrode 32.

Referring again to Figure 4, when CO₂ permeates the polymeric membrane 135 of the CO₂ electrode 40, it dissolves in the bicarbonate buffer of the inner solution layer 130 and changes the buffer pH, which changes the electrical potential of the CO₂ electrode 40. The inner solution layer of the pH electrode 32, however, is not affected by CO₂ in the analytical sample, so the pH electrode's potential remains constant. The difference in electrical potential between the CO₂ electrode 40 and the pH electrode 32 is proportional to the concentration of CO₂ in the sample. In one embodiment according to the invention, PVC-COOH can be a constituent of the polymeric membrane 135 of a CO₂ electrode 40.

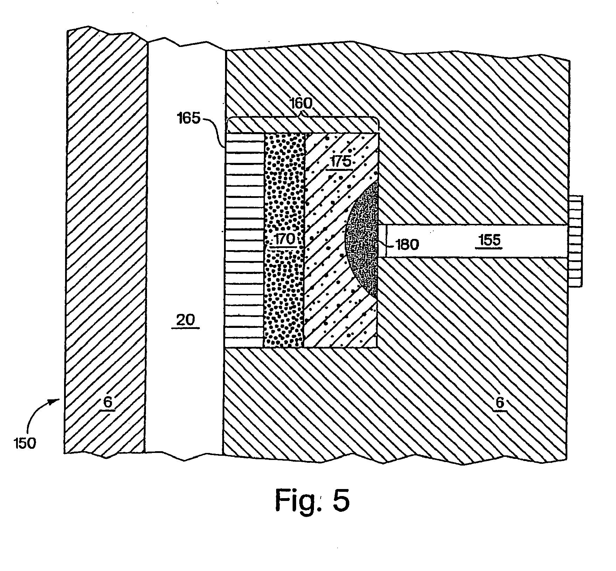

Figure 5 illustrates another embodiment according to the invention, an enzyme electrode 150 for detecting the presence and concentration of biological metabolites (such as a carbohydrate, peptide, lipid, nucleotide, or urea, for example) in an analytical sample. The enzyme electrode 150 comprises a metal element 155 embedded in an electrode card 6 and a composite membrane 160, which is located between the metal element 155 and an analytical sample flowing through a channel 20 in the electrode card 6. The composite membrane 160 includes an outer diffusional membrane 165 adjacent to the channel 20, an enzymatic layer 170, an ion-selective polymeric membrane 175, and an inner solution layer 180 adjacent to the metal element 155. The outer diffusional membrane 165 controls the diffusion of the analyte into the enzyme layer 170 and protects the other components of the enzyme electrode 150 from direct contact with the analytical sample in the channel 20. The enzyme layer 170 may include at least one enzyme, or a mixture of several enzymes, proteins, and stabilizers, that reacts with a particular analyte. If the analyte diffuses through the outer diffusional membrane 165, it can react with the enzyme(s) in the enzyme layer 170 to produce a chemical by product, which can migrate through the ion-selective polymeric membrane 175. In the case of a urea sensor, the chemical byproduct can be ammonium ions, for example. An electrical potential is generated across the composite membrane 160 that depends on the concentration of the chemical byproduct, which is proportional to the concentration of the analyte of interest in the analytical sample. In one embodiment according to the invention, PVC-COOH is a constituent of the ion-selective polymeric membrane 175.

In a particular embodiment, a urea sensor according to the invention includes an enzyme that catalyzes the hydrolysis of urea to ammonium ions, which are then detected by an ammonium ion-selective electrode. An example of a suitable enzyme is urease. Referring again to Figure 5, the composite membrane 160 of the urea sensor includes an outer diffusional membrane 165, an enzymatic layer 170 containing urease, an ammonium ion-selective polymeric membrane 175, and an inner solution layer 180 adjacent to a metal element 155.

The outer diffusional membrane 165 is formulated to serve several functions. First, the outer diffusional membrane 165 secures the enzymatic layer 170 to the ion-selective polymeric membrane 175, preventing the enzymatic layer 170 from being washed away by the flow of calibration solution or sample over the enzyme layer that would otherwise occur without the protective outer diffusional membrane 165. Second, the outer diffusional membrane 165 restricts the diffusion of contaminants from the sample into the enzymatic layer 170, advantageously extending the useful life of the sensor. In a particular embodiment, the outer diffusional membrane 165 is formulated to restrict the diffusion of urea into the enzymatic layer 170, thus allowing a smaller but proportional fraction of the urea in the sample to react with the enzyme. Restricting the amount of urea that reaches the enzymatic layer 170 not only extends the linear range of the urea sensor, but also inhibits the pH of the enzymatic layer 170 from increasing, which further extends the upper linear range of the sensor.

The outer diffusional membrane 165 generally includes one or more polymers. Suitable polymers include, but are not limited to, polyurethane, poly(tetrafluoroethylene) ionomers (*e.g*., the perfluorosulfonate ionomer sold under the tradename NAFION by E.I. Du Pont De Nemours & Co., Wilmington, DE), poly-(2-hydroxymethyl methacrylate), polyvinyl chloride, polyvinyl chloride derivatives (*e.g*., carboxylated polyvinyl chloride and hydroxylated polyvinyl chloride), polycarbonate, cellulose acetate, and mixtures and copolymers thereof. A preferred polymer for use in the outer diffusional membrane 165 is hydrophilic polyurethane (available, for example, from Thermedics, Inc., Woburn, MA). In one embodiment, the outer diffusional membrane 165 includes a mixture or co-polymer of two or more polymers. In another embodiment, the outer diffusional membrane 165 includes two or more distinct layers of identical or different polymers and/or identical or different co-polymers.

The enzymatic layer 170 includes an enzyme which catalyzes the reaction of urea to ammonium ions, such as, for example, urease. In one embodiment, the enzymatic layer 170 includes one or more enzyme stabilizers, including, for example, inert proteins (*e.g*., serum proteins, including bovine serum albumin), polypeptides (*e.g*., glutathione), and solvents. In a particular embodiment, glutathione is used together with one or more inert proteins to stabilize the urease in the enzyme layer 170. In addition, the urease may be cross-linked to glutathione and/or one or more inert proteins using a cross-linking agent (*e.g*., glutaraldehyde) to prevent migration of the enzyme and/or the stabilizers out of the enzymatic layer 170. Cross-linking also secures the enzymatic layer 170 to the underlying ion-selective polymeric layer 175. During fabrication of the enzymatic layer 170, the enzyme stabilizers are generally added to the solution containing the enzyme prior to the addition of the cross-linking agent to ensure the stabilizers are cross-linked together with the enzyme.

The ammonium ion-selective polymeric layer 175 includes an ammonium-selective ionophore disposed in a polymeric matrix. Suitable ionophores include, but are not limited to, nonactin, monactin, dinactin, trinactin, tetranactin, narasin, benzocrown ethers (see, *e.g*., Anal. Chem., 2000, 72, 4683-4688) cyclic depsipeptides (see, *e.g.,* Anal. Chem., 2003, 75, 152-156), and mixtures thereof. The polymeric matrix can include, for example, polyvinyl chloride, polyvinyl chloride derivatives (*e.g*., carboxylated polyvinyl chloride and hydroxylated polyvinyl chloride), polyurethane, poly(tetrafluoroethylene), poly(methyl methacrylate), silicone rubber, and mixtures and co-polymers thereof. In addition, the ammonium ion-selective polymeric layer 175 can contain other additives, such as plasticizers and lipophilic salt additives, as discussed herein.

Referring again to Figure 3, the polymeric membrane of an ISE regulates the selectivity of the ISE 100 toward an analyte of interest. The polymeric membrane 115 includes at least four elements: a polymer, a plasticizer, an ionophore, and a lipophilic salt additive.

In one embodiment according to the invention, PVC-COOH is a polymer component of the polymeric membrane 115. PVC-COOH is polyvinyl chloride (PVC) polymer that has a percentage of its chlorine atoms replaced by carboxyl groups (COOH). In an embodiment according to the invention, the PVC-COOH can contain between 0.1 and 5 % COOH by weight, and in a particular embodiment of the invention, the PVC-COOH contains 1.8 % COOH by weight. The PVC-COOH prevents lipophilic anionic species, (such as analgesics and anesthetics, for example) in the analytical sample from permeating the polymeric membrane 115 and interfering with the ISE 100. Such lipophilic anionic species can include thiopental sodium (thiopental), phenytoin, ibuprofen, fenoprofen, salicylate, valproate, and ε-amino-caproate, for example. PVC-COOH can be mixed with another polymer (such as HMW-PVC or polyurethane, for example) to form the polymer component of the polymeric membrane. In a particular embodiment of the invention, PVC-COOH is not mixed with another polymer and is the only polymer component of the polymeric membrane.

The polymeric membrane 115 that includes PVC-COOH also exhibits enhanced adhesive properties to solid platforms, which is important for the long life and potential stability of an electrochemical sensor. In addition, an electrochemical sensor employing PVC-COOH in its polymeric membrane 115 shows better potential stability and reproducibility of sensor measurements due to a significant reduction in the membrane resistance conferred by the relatively polar polymeric membrane 115, as illustrated by Example 6 below. The precision and accuracy of a PVC-COOH sensor is comparable to known ISEs, including sensors based on high molecular weight polyvinyl chloride (HMW-PVC), as illustrated by Example 7 below.

The plasticizer component of the polymeric membrane 115 provides ion mobility within the membrane that is necessary to obtain effective ion transfer. The plasticizer must be compatible with the polymer component and must be a solvent for the ionophore. The plasticizer must also be sufficiently insoluble in water so that it does not migrate significantly into an aqueous sample in contact with the surface of the polymeric membrane 115. It is also desirable that the plasticizer be substantially non-volatile to extend the shelf-life of the electrode. Useful plasticizers include bis(2-ethylhexyl) sebacate (DOS) and o-nitrophenyl octyl ether (NPOE).

The ionophore used in the polymeric membrane 115 is capable of selectively associating with a specific ion. This feature of the ionophore is responsible for the ion-selectivity of an ISE. Examples of suitable ionophores for a sodium ISE include methyl monensin ester, calixarene derivatives, and other sodium-sensitive compounds. A monocyclic antibiotic (such as valinomycin, for example) can be used as an ionophore for a potassium ISE. An ionophore for a calcium ISE may be, for example, (-)-(R,R)-N,N'-(Bis(11-ethoxycarbonyl)undecyl)-N,N'-4,5-tetramethyl-3,6-dioxaoctanediamide; Diethyl N,N'-[(4R,5R)-4,5-dimethyl-1,8-dioxo-3,6-dioxaoctamethylene]-bis(12-methylaminododecanoate) (ETH 1001). An example of a suitable ionophore for a pH electrode and/or a carbon dioxide electrode is tridodecylamine (TDDA).

The lipophilic salt additive used in the polymeric membrane 115 serves to reduce membrane resistance and to reduce anion interference. Useful lipophilic salt additives include, for example, potassium tetrakis(4-chlorophenyl)borate (KTpClPB) and potassium tetrakis[3,5-bis(trifluoromethyl)phenyl]borate (KTTFPB). The lipophilic salt additive, however, is not always essential to the function of the polymeric membrane 115 and can be omitted when certain analytes are targeted.

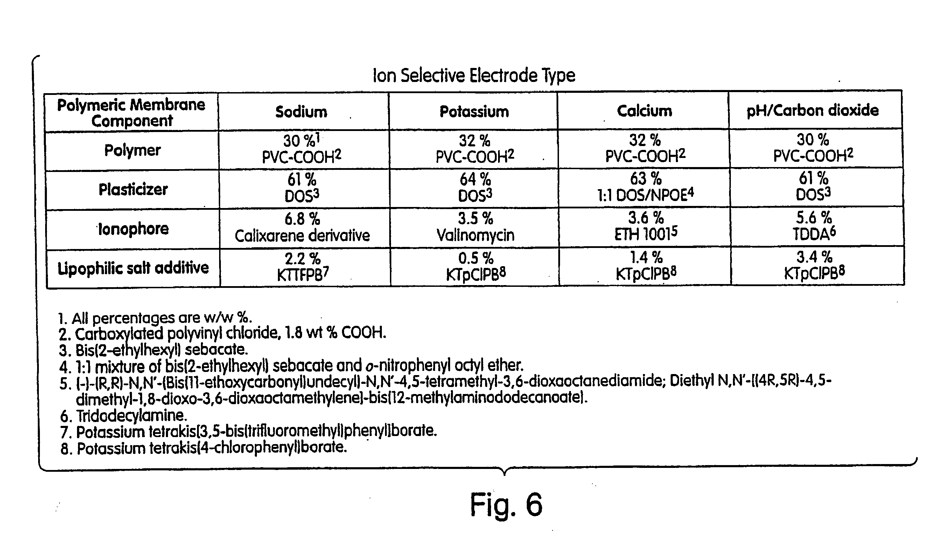

The efficiency of the polymeric membrane 115 in rejecting lipophilic anion drug contaminants is enhanced when the polymeric membrane 115 composition is optimized in terms of PVC-COOH/plasticizer ratio and by a proper selection of the type and ratios of ionophore and lipophilic salt additive. For example, the polymeric membrane 115 for a sodium ISE may contain 25-35 % PVC-COOH, 60-70 % DOS, 2-8 % calixarene derivative, and 1-3 % KTTFPB by weight. The polymeric membrane 115 for a potassium ISE may contain, for example, 25-35 % PVC-COOH, 60-70 % DOS, 1-5 % valinomycin, and 0-1 % KTpClPB by weight. The polymeric membrane 115 for a calcium ISE may contain, for example, 25-35 % PVC-COOH, 60-70 % 1:1 DOS/NPOE, 1-5 % ETH 1001, and 0.2-2 % KTpClPB by weight. The polymeric membrane 115 for a pH or CO₂ ISE may contain, for example, 25-35 % PVC-COOH, 60-70 % DOS, 2-7 % TDDA, and 1-4 % KTpClPB by weight. Figure 6 illustrates examples of particular embodiments of suitable polymeric membrane 115 components and their respective weight ratios for a variety of electrochemical sensors.

Referring still to Figure 3, a polymeric membrane 115 according to the present invention can be formed by dissolving the appropriate amounts of polymer, plasticizer, ionophore, and lipophilic salt additive in a solvent, typically tetrahydrofuran (THF) or cyclohexanone, and applying this solution to the exposed surface of a metal element 105 embedded in an electrode card 6. For example, a potassium ISE can be fabricated by mixing PVC-COOH (1.8 wt % COOH), DOS, valinomycin and KTpClPB according to the ratios listed in Figure 6 to make a total mass of 630-650 mg. The mixture is dissolved in 3-3.5 mL THF, and 0.75 µL of the solution is applied to the exposed end of the metal element 105 (for example, a chloridized silver wire) embedded in the electrode card 6. Once the solvent evaporates, the same volume of membrane solution is applied two additional times with adequate drying time in between each application. Once the solvent has evaporated from the last application, the polymeric membrane 115 is formed and is bonded to the electrode card 6.

Referring again to Figure 5, in one embodiment, a urea sensor according to the present invention is fabricated by first applying the inner solution layer 180 (*e.g*., a MES buffer solution) to the exposed surface of a metal element 155 (*e.g*., silver/silver chloride or platinum) embedded in an electrode card 6. Next, a solution of nonactin, polyvinyl chloride, and one or more plasticizers is added over the inner solution layer 180 to form the ion-selective polymer layer 175. The enzyme layer 170 is formed by adding a solution urease, glutathione, bovine serum albumin, and glutaraldehyde to the surface of the ion-selective polymer layer 175. Finally, a solution of hydrophilic polyurethane is added over the enzyme layer 170 to form the outer diffusional layer 165.

An ISE according to the present invention measures changes in the electric potential, measured in millivolts (mV), of an analytical sample that are due to changes in the concentrations of analytes within the sample. Similarly, a gas electrode according to the present invention measures changes in the electric potential of an analytical sample that are due to changes in the partial pressure of the gas dissolved in the sample. As practitioners skilled in the art are aware, electric potential values are related to concentration or partial pressure values according to the Nernst equation. In a particular embodiment according to the invention, software may be included in the electrochemical sensor system to convert electrical potential values measured by the electrode to concentration or partial pressure values of the measured analyte by using the Nernst equation.

In another aspect, the invention is a method for detecting the presence and/or measuring the concentration of an analyte in a body fluid (such as blood, for example) in the presence of a lipophilic anionic species (such as thiopental, for example) without interference by the lipophilic anionic species in detecting and/or measuring the analyte. The method of the invention provides an electrochemical sensor for detecting and/or measuring an analyte of interest in a body fluid that includes PVC-COOH as a polymer component of the sensor's polymeric membrane. A body fluid sample containing the analyte of interest and a lipophilic anionic contaminant is placed in contact with the electrochemical sensor that includes PVC-COOH as a polymer component of the sensor's polymeric membrane. The analyte of interest in the body fluid sample is then measured and/or detected by the electrochemical sensor with reduced interference by the lipophilic anionic contaminant.

The following examples are intended to illustrate, but not limit, the invention.

### Example 1

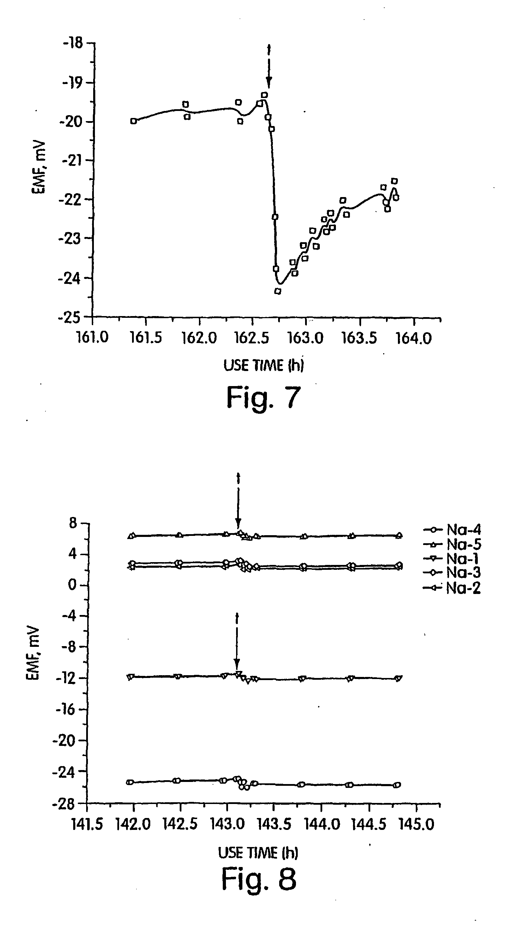

Figure 7 is a graphical representation of the chronopotentiometric responses recorded from an electrode card including a sodium ISE with a polymeric membrane containing HMW-PVC. An analytical sample containing a known concentration of sodium was introduced to the electrode card, and the ISE measured the concentration of sodium to be 142 mM. At time t, the analytical sample was changed to a solution containing the same concentration of sodium plus 10 mg/dL thiopental, and the ISE returned a sodium concentration value of 137 mM.

Figure 8 is a graphical representation of the chronopotentiometric responses recorded from an electrode card including five sodium ISEs with polymeric membranes containing PVC-COOH according to the invention. An analytical sample containing a known concentration of sodium was introduced to the electrode card, and all five ISEs measured the concentration of sodium to be 139 mM. At time t, the analytical sample was changed to a solution containing the same concentration of sodium plus 10 mg/dL thiopental, and all five electrodes returned sodium concentration values of 139 mM. The PVC-COOH based sodium ISEs did not exhibit the drift in potential displayed by the HMW-PVC based sodium ISE after analyzing a sample contaminated with thiopental, which illustrates the efficacy of PVC-COOH in preventing interference with sodium measurements caused by lipophilic anionic contaminants.

### Example 2

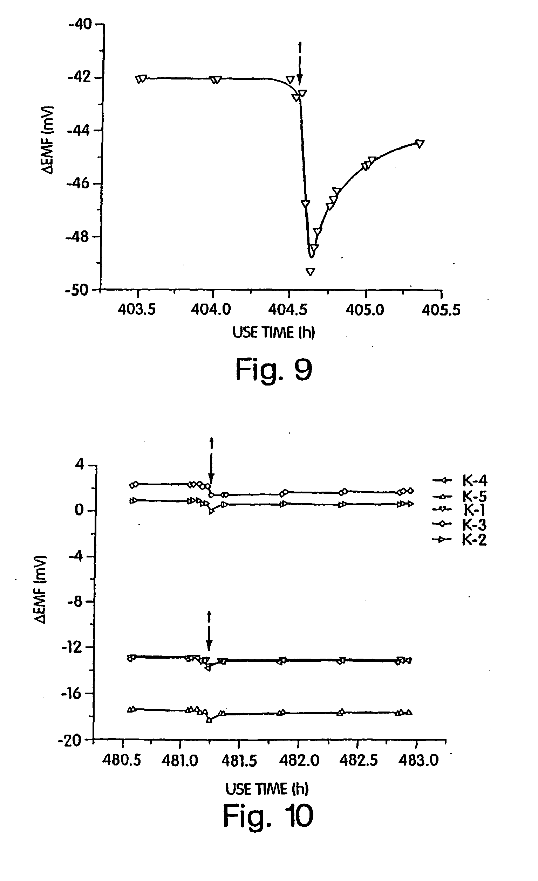

Figure 9 is a graphical representation of the chronopotentiometric responses recorded from an electrode card including a potassium ISE with a polymeric membrane containing HMW-PVC. An analytical sample containing a known concentration of potassium was introduced to the electrode card, and the ISE measured the concentration of potassium to be 3.2 mM. At time t, the analytical sample was changed to a solution containing the same concentration of potassium plus 10 mg/dL thiopental, and the ISE returned a potassium concentration value of 2.8 mM.

Figure 10 is a graphical representation of the chronopotentiometric responses recorded from an electrode card including five potassium ISEs with polymeric membranes containing PVC-COOH according to the invention. An analytical sample containing a known concentration of potassium was introduced to the electrode card, and all five ISEs measured the concentration of potassium to be 3.3 mM. At time t, the analytical sample was changed to a solution containing the same concentration of potassium plus 10 mg/dL thiopental, and all five electrodes returned potassium concentration values of 3.3 mM. The PVC-COOH based potassium ISEs exhibited negligible potential drifts after analyzing a sample contaminated with thiopental as compared to the drift displayed by the HMW-PVC based potassium ISE, which illustrates the efficacy of PVC-COOH in preventing interference with potassium measurements caused by lipophilic anionic contaminants.

### Example 3

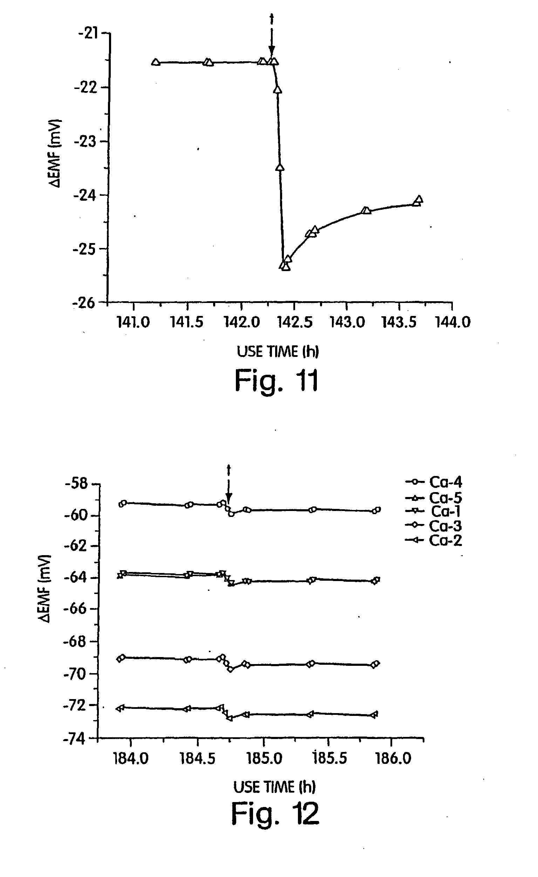

Figure 11 is a graphical representation of the chronopotentiometric responses recorded from an electrode card including a calcium ISE with polymeric membrane containing HMW-PVC. An analytical sample containing a known concentration of calcium was introduced to the electrode card, and the ISE measured the concentration of calcium to be 0.93 mM. At time t, the analytical sample was changed to a solution containing the same concentration of calcium plus 10 mg/dL thiopental, and the ISE returned a calcium concentration value of 0.81 mM.

Figure 12 is a graphical representation of the chronopotentiometric responses recorded from an electrode card including five calcium ISEs with polymeric membranes containing PVC-COOH according to the invention. An analytical sample containing a known concentration of calcium was introduced to the electrode card, and all five ISEs measured the concentration of calcium to be 0.87 mM. At time t, the analytical sample was changed to a solution containing the same concentration of calcium plus 10 mg/dL thiopental, and the five electrodes returned calcium concentration values of 0.85, 0.85, 0.86, 0.86, and 0.85 mM. The PVC-COOH based calcium ISEs did not exhibit as great a drift in potential as displayed by the HMW-PVC based calcium ISE after analyzing a sample contaminated with thiopental, which illustrates the efficacy of PVC-COOH in preventing interference with calcium measurements caused by lipophilic anionic contaminants.

### Example 4

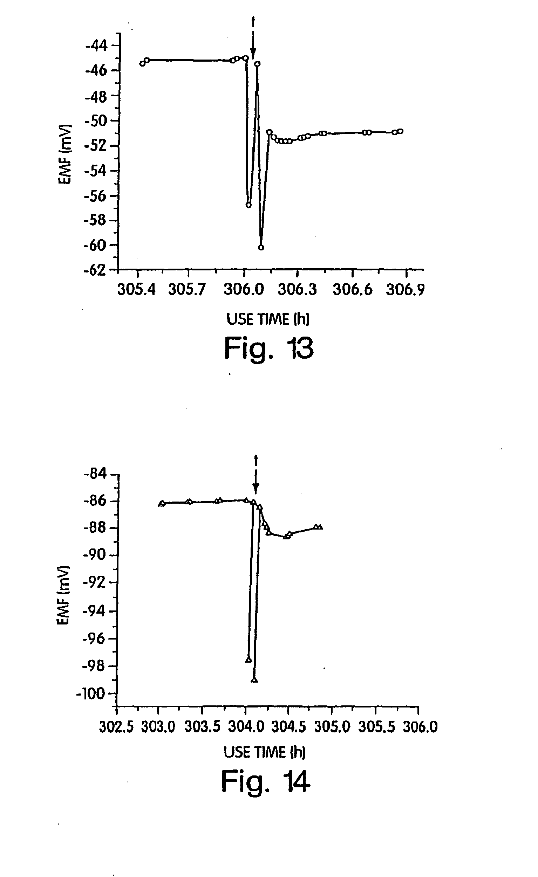

Figure 13 is a graphical representation of the chronopotentiometric responses recorded from an electrode card including a pH electrode with a polymeric membrane containing HMW-PVC. An analytical sample containing a buffer solution of known pH was introduced to the electrode card, and the electrode measured the pH to be 7.63. At time t, the analytical sample was changed to a solution containing the same buffer solution plus 10 mg/dL thiopental, and the electrode returned a pH value of 7.68.

Figure 14 is a graphical representation of one of the chronopotentiometric responses recorded from an electrode card including five pH electrodes with polymeric membranes containing PVC-COOH according to the invention. An analytical sample containing a buffer solution of known pH was introduced to the electrode card, and all five electrodes measured the pH to be 7.66. At time t, the analytical sample was changed to a solution containing the same buffer solution plus 10 mg/dL thiopental, and the five electrodes returned pH values of 7.68. The PVC-COOH based pH electrodes did not exhibit as great a drift in potential as displayed by the HMW-PVC based pH electrode after analyzing a sample contaminated with thiopental, which illustrates the efficacy of PVC-COOH in preventing interference with pH measurements caused by lipophilic anionic contaminants.

### Example 5

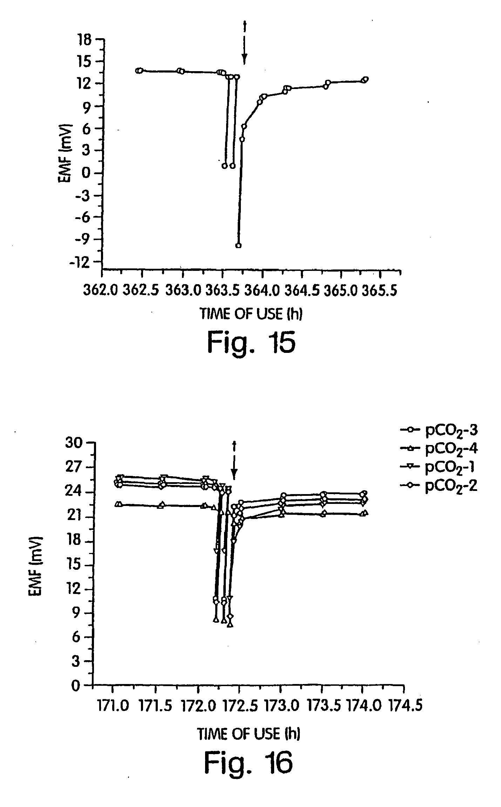

Figure 15 is a graphical representation of the chronopotentiometric responses recorded from an electrode card including a CO₂ electrode with polymeric membrane containing HMW-PVC. An analytical sample containing a buffer solution was introduced to the electrode card. Two measurements of the partial pressure of CO₂ in the solution were taken, and the results were averaged to yield a value of 66 mm Hg. At time t, the analytical sample was changed to a solution containing the same buffer solution plus 10 mg/dL thiopental, and the electrode returned CO₂ partial pressure value of 115 mm Hg.

Figure 16 is a graphical representation of the chronopotentiometric responses recorded from an electrode card including four CO₂ electrodes with polymeric membranes containing PVC-COOH according to the invention. An analytical sample containing a buffer solution was introduced to the electrode card. Two measurements of the partial pressure of CO₂ in the solution were taken with each of the four electrodes, and the results for each electrode were averaged to yield values of 67.9, 68.0, 68.0, and 68.2 mm Hg. At time t, the analytical sample was changed to a solution containing the same buffer solution plus 10 mg/dL thiopental, and the four electrodes returned CO₂ partial pressure values of 70.4, 68.8, 68.8, and 73.4 mm Hg. The PVC-COOH based CO₂ electrodes display negligible potential drifts after analyzing a sample contaminated with thiopental as compared to the drift displayed by the HMW-PVC based CO₂ electrode, which illustrates the efficacy of PVC-COOH in preventing interference with CO₂ measurements caused by lipophilic anionic contaminants.

### Example 6

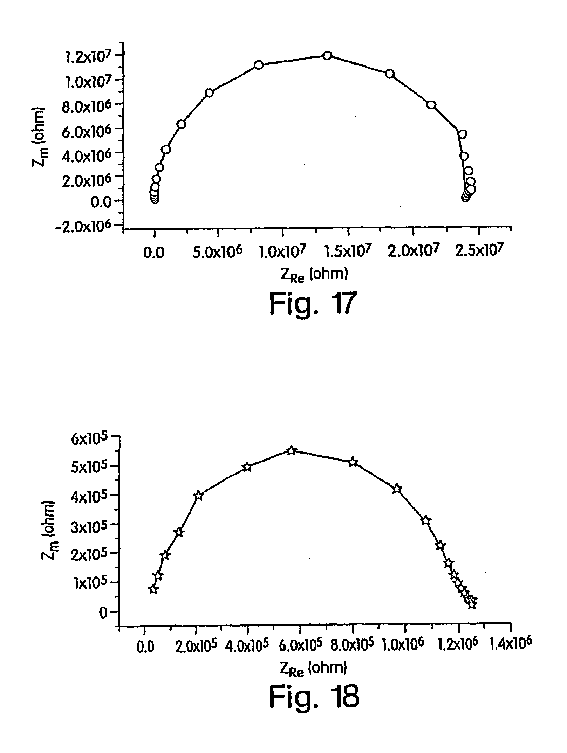

Figure 17 is a graphical representation of the bulk membrane resistance of an ISE polymeric membrane that includes HMW-PVC as a polymer component, as is known in the art. According to Figure 17, the HMW-PVC membrane has a bulk resistance of about 2.4 x 10⁷ ohms. By comparison, Figure 18 shows that the bulk membrane resistance of an ISE polymeric membrane that includes PVC-COOH as a polymer component according to the invention is about 1.25 x 10⁶ ohms, which is over nineteen times lower than that of an ISE containing HMW-PVC. By lowering the bulk membrane resistance, an ISE fabricated using PVC-COOH in its polymeric membrane exhibits enhanced potential stability and reproducibility of measurements.

### Example 7

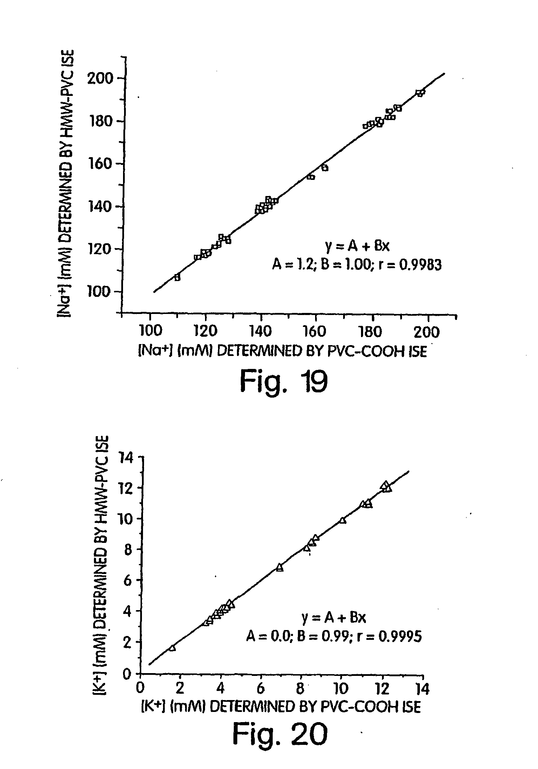

Figure 19 is a graphical representation comparing sodium concentration values in whole blood samples determined by a known sodium ISE with a polymeric membrane containing HMW-PVC against those determined by an electrode card including five sodium ISEs with polymeric membranes containing PVC-COOH according to the invention. Sodium concentration values for ninety-nine different whole blood samples representing a wide range of sodium levels were first determined with a HMW-PVC based sodium ISE, then with a PVC-COOH based sodium ISE. As illustrated by Figure 19, the values obtained from the PVC-COOH based sodium ISE correlate well with those obtained using the HMW-PVC based sodium ISE (r = 0.9983), which indicates the PVC-COOH based sodium ISE measures sodium with equal precision and accuracy as a known sodium electrode.

Figure 20 illustrates a similar experiment using a PVC-COOH based potassium ISE. Potassium concentration values for 127 different whole blood samples representing a wide range of potassium levels were first determined using a HMW-PVC based potassium ISE, then a PVC-COOH based potassium ISE. As illustrated by Figure 20, the values obtained from the PVC-COOH based potassium ISE correlate well with those obtained using the HMW-PVC based electrode (r = 0.9995), which indicates that the PVC-COOH based potassium ISE measures potassium with equal precision and accuracy as a known potassium electrode.

### Example 8

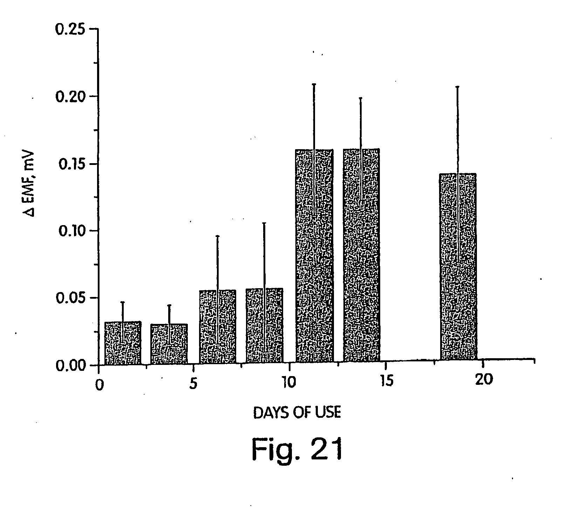

Figure 21 is a graphical representation of the stability over time of a PVC-COOH based sodium ISE according to the invention. On day 1, an analytical sample containing a known concentration of sodium was introduced to an electrode card including five sodium ISEs with polymeric membranes containing PVC-COOH according to the invention, and the concentration of sodium was measured. Then the analytical sample was changed to a solution containing the same concentration of sodium plus 10 mg/dL thiopental, and the concentration of sodium was measured again. The difference between the two measurements (ΔEMF), which represents the drift in the electrode's potential due to thiopental interference, was calculated, and the analytical sample was removed from the sensor card. This procedure was repeated on days 2, 4, 6, 9, 12, 14, and 20, and the ΔEMF values were calculated. As Figure 21 illustrates, the effect that a lipophilic anionic species such as thiopental has on a PVC-COOH based sodium ISE according to the invention remains very low throughout the life of the electrode.

### Example 9

A urea sensor was fabricated according to the invention as follows. A MES buffer solution was prepared containing 50.9 mmol/L MES potassium salt, 46.6 mmol/L MES free acid, and 2 mmol/L potassium chloride, and 0.05 µL of this solution was applied to the end of a silver/silver chloride wire embedded in a PVC electrode card. Next, an ion-selective membrane solution was prepared by combining 150 mg PVC, 100 µL dioctyl sebacate, 100 µL 2-nitrophenyl octyl ether, 110 µL of 1% potassium tetrakis(4-chlorophenyl)borate in THF, and 300 µL of 5% nonactin in THF in 8 mL THF. Two 0.75 µL aliquots of this solution were applied sequentially over the MES buffer layer to form the ion-selective membrane. Next, an enzyme solution was prepared containing 50 mg/mL urease (Biozyme, San Diego, CA), 20 mg/mL glutathione, 10 mg/mL bovine serum albumin, and 0.12% glutaraldehyde in 0.1 M phosphate buffer at a pH of 7.2, and 0.075 µL of this solution was applied to the ion-selective membrane. Finally, an outer membrane solution was prepared containing 0.12 g/ml polyurethane (Thermedics, Inc., Woburn, MA) in THF, and 0.5 µL of this solution was applied over the enzyme layer to form the outer membrane. The urea sensor was assembled into a disposable cartridge and tested on a GEM Premier 3000 analyzer (Instrumentation Laboratory Company, Lexington, MA).

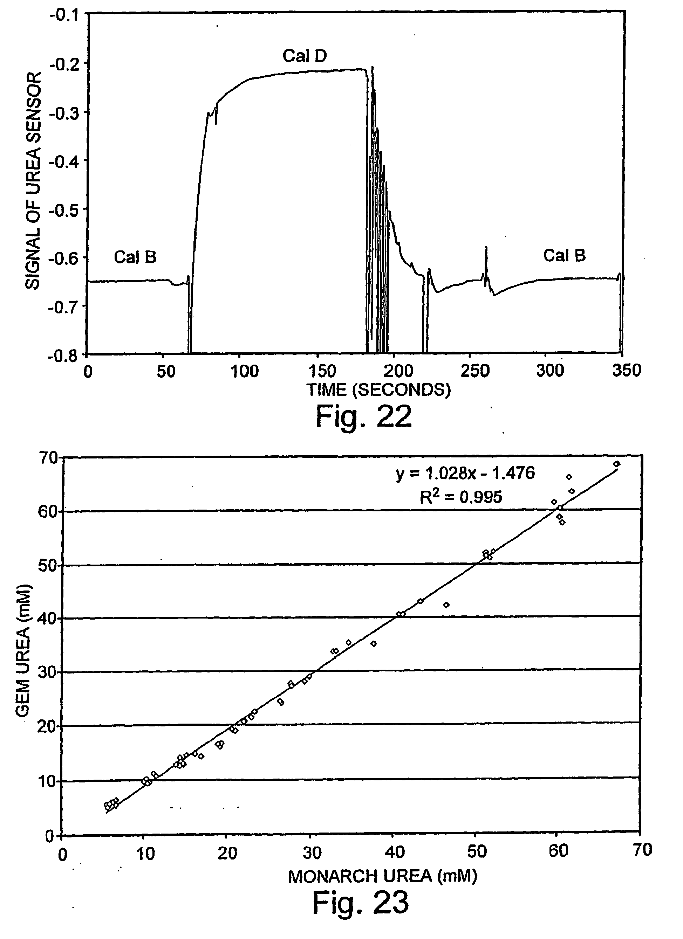

During use, urea sensors according to the invention are automatically calibrated using calibration solutions enclosed in the disposable cartridge according to a predetermined schedule throughout the useful life of the sensor. Calibration is performed to determine the sensitivity of the urea sensor to urea (*i.e*., the slope of the urea response curve) and the sensor's selectivity coefficient for urea over potassium. To determine the sensitivity of urea sensors according to the invention, the urea sensor described above was calibrated with two different calibration solutions. The first calibration solution (Cal B), which contained 1.0 mmol/L urea and 2.0 mmol/L potassium, was introduced into the sample channel of the cartridge, and the signal of the urea sensor was recorded. Next, Cal B was replaced with the second calibration solution (Cal D), which contained 7.5 mmol/L urea and 15 mmol/L potassium, and the sensor response was recorded. Finally, the calibration solution was switched back to Cal B, and the sensor response was recorded again. The sensor is always soaked in the Cal B solution, except during calibration and sample analysis. As shown in Figure 22, the urea sensor responded rapidly and in a stable manner to changes in the urea and potassium concentrations, which indicates that urea sensors according to the invention are capable of rapid analysis of analytical samples.

Next, the analytical performance of the urea sensor was evaluated by analyzing the urea concentration of sixty-two whole blood samples tested over a period of three weeks. The blood samples were tested on the fourth, ninth, fourteenth, and twenty-second days of the study. For each test, heparinized whole blood samples were collected from healthy individuals and spiked with a concentrated urea solution to yield samples with urea concentrations ranging from 5 to 70 mmol/L. The urea concentration value of each sample then was determined using a urea sensor according to the invention as described above. For comparison, each aliquot was centrifuged after analysis to isolate the plasma, and the plasma urea concentration was determined using a conventional clinical chemistry analyzer (MONARCH, Instrumentation Laboratory Company, Lexington, MA). The Monarch analyzer uses a spectrophotometric method in combination with a bienzymatic reaction of urease, glutamate dehydrogenase, and NADH to detect urea concentration in a sample. Because the Monarch analyzer can only measure up to 35 mmol/L urea, the plasma samples were diluted with saline prior to analysis. The results of the experiments are summarized in Figure 23.

As Figure 23 indicates, the urea concentration values obtained from the urea sensor of the present invention correlate well with those obtained using the Monarch analyzer (r = 0.995), which indicates the urea sensor of the present invention measures urea with equal precision and accuracy as a conventional chemistry analyzer. Urea sensors according to the present invention have the added benefit of being less expensive and easier to use than the Monarch analyzer. In addition, the experiments indicate that the urea sensor has a useful life of at least three weeks.

### Example 10

Ammonium ion-selective membranes of urea sensors according to the invention can be at least partially permeable to potassium ions, which can lead to erroneous urea concentration values. One method for correcting the urea concentration value for potassium interference involves independently measuring the potassium concentration in the sample (using, for example, a potassium ISE as described herein), determining a correction factor based on the potassium concentration and the selectivity coefficient of the urea sensor for urea over potassium, and then subtracting the correction factor from the measured urea concentration value to obtain the actual urea concentration value of the sample.

To determine if potassium interference can be corrected for using urea sensors according to the invention, two urea sensors were fabricated according to the method described in Example 9 and incorporated into a disposable cartridge that also contained a potassium ISE. The selectivity coefficient of each urea sensor was determined by calibrating with two calibration solutions: Cal A (1.0 mmol/L urea, 7.5 mmol/L potassium) and Cal B (1.0 mmol/L urea, 2.0 mmol/L potassium). Five whole blood samples were spiked with different amounts of potassium, yielding samples with potassium concentrations that ranged from 8 to 24 mmol/L. An un-spiked sample had a potassium concentration of 4.2 mmol/L and a urea concentration of 5.0 mmol/L. Each sample was introduced to the cartridge, and the potassium and urea concentration values were recorded. The urea concentration values were corrected based on the selectivity coefficient and the potassium concentrations. The results of the experiments are summarized in Table 1, below.

**Table 1**

| **Potassium Concentration (mmol/L)** | **Corrected Urea Concentration, Sensor 1 (mmol/L)** | **Corrected Urea Concentration, Sensor : (mmol/L)** |
|---|---|---|
| 4.2 | 5.0 | 5.0 |
| 8.5 | 5.0 | 5.0 |
| 12.1 | 5.0 | 5.0 |
| 16.0 | 5.0 | 5.0 |
| 20.0 | 5.0 | 5.0 |
| 24.3 | 5.0 | 5.0 |

As Table 1 indicates, potassium interference can effectively be eliminated for urea sensors according to the present invention.

Variations, modifications, and other implementations of what is described herein will occur to those of ordinary skill in the art without departing from the scope of the invention as claimed. Accordingly, the invention is to be 5 defined not by the preceding illustrative description but instead by the scope of the following claims.

## Claims

1. A sensor for detecting urea in a sample, comprising:
an electrode;
an ion-selective membrane comprising a polymer matrix comprising an ammonium-ion selective ionophore;
an enzymatic layer disposed on the surface of the ion-selective membrane, said enzymatic layer comprising urease; and
an outer diffusional layer for contacting a sample, wherein the outer diffusional layer is disposed on the enzymatic layer, is permeable to urea, and comprises hydrophilic polyurethane.

2. The sensor of claim 1, wherein the electrode comprises a metal electrode; and in which case optionally wherein the electrode comprises a silver/silver chloride electrode.

3. The sensor of claim 1, wherein the enzymatic layer:
(a) catalyzes the hydrolysis of urea to ammonium ions; or
(b) further comprises one or more enzyme stabilizers; and in which case optionally wherein the enzyme stabilizer is selected from the group consisting of polypeptides and inert proteins; and in which optionally either: wherein the polypeptide comprises glutathione, or wherein the inert protein comprises bovine serum albumin; or
(c) comprises urease cross-linked to glutathione; and in which case optionally further comprising one or more inert proteins cross-linked to at least one of the urease and the glutathione; and in which case optionally wherein the inert protein comprises bovine serum albumin.

4. The sensor of claim 1, wherein the polymer matrix is selected from the group consisting of polyvinyl chloride, carboxylated polyvinyl chloride, hydroxylated polyvinyl chloride, polyurethane, poly(tetrafluoroethylene), poly(methyl methacrylate), silicone rubber, and mixtures thereof.

5. The sensor of claim 1, wherein the ionophore is selected from the group consisting of nonactin, monactin, dinactin, trinactin, tetranactin, narasin, benzocrown ethers, cyclic depsipeptides, and mixtures thereof.

6. The sensor of claim 1, wherein the sample is selected from the group consisting of blood, serum, plasma, cerebro-spinal fluid, saliva, and urine.

7. A sensor card comprising:
a substrate; and
one or more sensors of claim 1 disposed on or in the substrate.

8. A method of forming the sensor of claim 1, the method comprising the steps of:
providing an electrode;
applying an inner solution layer to the electrode;
applying over the inner solution layer an ion-selective membrane;
applying to the ion-selective membrane an enzymatic layer; and
applying an outer diffusional layer over the enzymatic layer,
wherein the sensor detects or measures urea in a sample.

9. A sensor for detecting urea in a sample comprising:
an electrode and a composite membrane, said composite membrane comprising
an inner solution layer adjacent to the electrode;
an ion-selective membrane positioned next to the inner solution layer, said ion-selective membrane comprising a polyvinylchloride matrix and nonactin;
an enzymatic layer disposed on the surface of the ion-selective membrane, said enzymatic layer comprising glutaraldehyde, and urease cross-linked to glutathione; and
an outer diffusional layer permeable to urea and disposed on the enzymatic layer, wherein the outer diffusional layer is for contacting a sample and comprises hydrophilic polyurethane.

## Patentansprüche

1. Ein Sensor zum Nachweis von Harnstoff in einer Probe, umfassend:
eine Elektrode;
eine ionenselektive Membran, umfassend eine Polymermatrix, umfassend einen Ammoniumionen-selektiven lonophor;
eine Enzymschicht, die auf der Oberfläche der ionenselektiven Membran angeordnet ist, wobei besagte Enzymschicht Urease umfasst; und
eine äußere Diffusionsschicht zum Inkontaktbringen einer Probe, wobei die äußere Diffusionsschicht auf der Enzymschicht angeordnet ist, für Harnstoff durchlässig ist und hydrophiles Polyurethan umfasst.

2. Der Sensor nach Anspruch 1, wobei die Elektrode eine Metallelektrode umfasst; und wobei in diesem Fall gegebenenfalls die Elektrode eine Silber/Silberchlorid-Elektrode umfasst.

3. Der Sensor nach Anspruch 1, wobei die Enzymschicht:
(a) die Hydrolyse von Harnstoff zu Ammoniumionen katalysiert; oder
(b) außerdem einen oder mehrere Enzymstabilisatoren umfasst; und wobei in diesem Fall gegebenenfalls der Enzymstabilisator aus der Gruppe ausgewählt ist, bestehend aus Polypeptiden und inerten Proteinen; und wobei gegebenenfalls entweder das Polypeptid Glutathion umfasst oder das inerte Protein bovines Serumalbumin umfasst; oder
(c) Urease vernetzt mit Glutathion umfasst; und wobei in diesem Fall gegebenenfalls außerdem ein oder mehrere inerte Proteine wenigstens mit Urease oder Glutathion vernetzt sind; und wobei in diesem Fall gegebenenfalls das inerte Protein bovines Serumalbumin umfasst.

4. Der Sensor nach Anspruch 1, wobei die Polymermatrix aus der Gruppe ausgewählt ist, bestehend aus Polyvinylchlorid, carboxyliertem Polyvinylchlorid, hydroxyliertem Polyvinylchlorid, Polyurethan, Poly(tetrafluorethylen), Poly(methylmethacrylat), Silicongummi und Gemischen davon.

5. Der Sensor nach Anspruch 1, wobei der lonophor aus der Gruppe ausgewählt ist, bestehend aus Nonactin, Monactin, Dinactin, Trinactin, Tetranactin, Narasin, Benzokronenethern, cyclischen Depsipeptiden und Gemischen davon.

6. Der Sensor nach Anspruch 1, wobei die Probe aus der Gruppe ausgewählt ist, bestehend aus Blut, Serum, Plasma, Cerebrospinalflüssigkeit, Speichel und Urin.

7. Eine Sensorkarte, umfassend:
ein Substrat; und
einen oder mehrere Sensoren nach Anspruch 1, der/die auf oder in dem Substrat angeordnet ist/sind.

8. Ein Verfahren zur Bildung des Sensors nach Anspruch 1, wobei das Verfahren die Schritte umfasst:
Bereitstellen einer Elektrode;
Auftragen einer inneren Lösungsschicht auf die Elektrode;
Auftragen einer ionenselektiven Membran über die innere Lösungsschicht;
Auftragen einer Enzymschicht auf die ionenselektive Membran; und
Auftragen einer äußeren Diffusionsschicht über die Enzymschicht,
wobei der Sensor Harnstoff in einer Probe nachweist oder misst.

9. Ein Sensor zum Nachweis von Harnstoff in einer Probe, umfassend:
eine Elektrode und eine Kompositmembran, wobei besagte Kompositmembran umfasst
eine innere Lösungsschicht, die an die Elektrode grenzt;
eine ionenselektive Membran, die der inneren Lösungsschicht nächst positioniert ist, wobei besagte ionenselektive Membran eine Polyvinylchloridmatrix und Nonactin umfasst;
eine Enzymschicht, die auf der Oberfläche der ionenselektiven Membran angeordnet ist, wobei besagte Enzymschicht Glutaraldehyd und mit Glutathion vernetzte Urease umfasst; und
eine äußere Diffusionsschicht, die für Harnstoff durchlässig ist und auf der Enzymschicht angeordnet ist, wobei die äußere Diffusionsschicht für einen Kontakt mit einer Probe dient und hydrophiles Polyurethan umfasst.

## Revendications

1. Capteur pour détecter l'urée dans un échantillon, comprenant :
une électrode;
une membrane sélective à l'égard des ions comprenant une matrice polymère comprenant un ionophore sélectif à l'égard des ions ammonium;
une couche enzymatique disposée sur la surface de la membrane sélective à l'égard des ions, ladite couche enzymatique comprenant de l'uréase; et
une couche de diffusion externe pour entrer en contact avec un échantillon, où la couche de diffusion externe est disposée sur la couche enzymatique, est perméable à l'urée et comprend un polyuréthane hydrophile.

2. Capteur selon la revendication 1 où l'électrode comprend une électrode métallique; et auquel cas éventuellement où l'électrode comprend une électrode à l'argent/chlorure d'argent.

3. Capteur selon la revendication 1 où la couche enzymatique:
(a) catalyse l'hydrolyse de l'urée en ions ammonium; ou
(b) comprend en outre un ou plusieurs stabilisants d'enzyme; et auquel cas éventuellement où le stabilisant d'enzyme est choisi dans le groupe consistant en les polypeptides et les protéines inertes; et/où éventuellement: où le polypeptide contient du glutathion, ou bien où la protéine inerte comprend de la sérumalbumine bovine; ou
(c) comprend de l'uréase pontée au glutathion; et auquel cas éventuellement comprenant en outre une ou plusieurs protéines inertes pontées à au moins l'un de l'uréase et du glutathion; et auquel cas éventuellement où la protéine inerte comprend de la sérumalbumine bovine.

4. Capteur selon la revendication 1 où la matrice polymère est choisie dans le groupe consistant en le poly(chlorure de vinyle), le poly(chlorure de vinyle) carboxylé, le poly(chlorure de vinyle) hydroxylé, un polyuréthane, le poly(tétrafluoroéthylène), le poly(méthacrylate de méthyle), un caoutchouc de silicone, et leurs mélanges.

5. Capteur selon la revendication 1 où l'ionophore est choisi dans le groupe consistant en la nonactine, la monactine, la dinactine, la trinactine, la tétranactine, la narasine, les éthers benzocouronnes, les depsipeptides cycliques et leurs mélanges.

6. Capteur selon la revendication 1 où l'échantillon est choisi dans le groupe consistant en le sang, le sérum, le plasma, le liquide céphalorachidien, la salive et l'urine.

7. Carte de capteur comprenant:
un substrat; et
un ou plusieurs capteurs selon la revendication 1 disposés sur ou dans le substrat.

8. Procédé de formation du capteur selon la revendication 1, le procédé comprenant les étapes de:
fournir une électrode;
appliquer une couche de solution interne à l'électrode;
appliquer sur la couche de solution interne une membrane sélective à l'égard des ions;
appliquer à la membrane sélective à l'égard des ions une couche enzymatique; et
appliquer une couche de diffusion externe sur la couche enzymatique,
où le capteur détecte ou mesure l'urée dans un échantillon.

9. Capteur pour détecter l'urée dans un échantillon comprenant:
une électrode et une membrane composite, ladite membrane composite comprenant
une couche de solution interne adjacente à l'électrode ;
une membrane sélective à l'égard des ions positionnée à proximité de la couche de solution interne, ladite membrane sélective à l'égard des ions comprenant une matrice de poly(chlorure de vinyle) et de la nonactine ;
une couche enzymatique disposée sur la surface de la membrane sélective à l'égard des ions, ladite couche enzymatique comprenant du glutaraldéhyde, et de l'uréase pontée au glutathion; et
une couche de diffusion externe perméable à l'urée et disposée sur la couche enzymatique,
où la couche de diffusion externe est destinée à entrer en contact avec un échantillon et comprend un polyuréthane hydrophile.
